Europäisches Patentamt

European Patent Office

Office européen des brevets

(19)

(11) Numéro de publication: **0 147 699**
**B1**

(12) # FASCICULE DE BREVET EUROPEEN

(45) Date de publication du fascicule du brevet:
**15.07.87**

(51) Int. Cl.⁴: **A 23 L 1/305**, A 61 K 31/405

(21) Numéro de dépôt: **84114956.0**

(22) Date de dépôt: **08.12.84**

(54) **Produit alimentaire efficace dans le traitement de la lépre.**

(30) Priorité: **04.01.84 CH 26/84**

(43) Date de publication de la demande:
**10.07.85 Bulletin 85/28**

(45) Mention de la délivrance du brevet:
**15.07.87 Bulletin 87/29**

(84) Etats contractants désignés:
**CH FR GB LI**

(56) Documents cités:
**EP - A - 0 053 985**
**DE - A - 2 829 036**
**FR - A - 907 314**
**FR - A - 1 162 773**
**FR - A - 2 154 397**
**FR - A - 2 332 027**
**US - A - 4 112 123**

**CHEMICAL ABSTRACTS, vol. 100, no. 9, 27 février 1984,**
**page 355, réf. no. 74005k, Columbus, Ohio, US**
**DICTIONNAIRE VIDAL, 1983, page 770, OVP, Paris, FR**

(73) Titulaire: **SOCIETE DES PRODUITS NESTLE S.A., Case**
**postale 353, CH-1800 Vevey (CH)**

(72) Inventeur: **Mauron, Jean, Avenue de Blonay 2,**
**CH-1800 Vevey (CH)**
Inventeur: **Mester de Parajd, Lazlo, Parc de Béarn 3,**
**F-92210 Saint-Cloud (FR)**

## Description

L'invention concerne le domaine de la nutrition thérapeutique. Elle a trait à un produit alimentaire efficace dans le traitement de la lèpre.

On a constaté depuis longtemps que les corps gras interféraient avec la prolifération de la lèpre: par exemple l'huile de Chaulmoogra, dont les acides gras en $C_{16}$ et en $C_{18}$ contiennent un radical cyclopenténylе, a montré une faible activité dans le traitement de cette maladie. D'autre part, les acides insaturés oxydés, par exemple ceux contenus dans les huiles de poisson rances, semblent favoriser la prolifération de la lèpre comme l'indique la prépondérance historique de la lèpre en Norvège. Mais faute d'avoir montré une efficacité suffisante, l'approche diététique a été abandonnée au profit de la voie pharmacologique. Ainsi la lèpre est principalement traitée par un médicament à base de 4,4'-diamino-diphénylsulfone (DDS). Cependant on a constaté que dans un certain nombre de cas le bacille responsable de la maladie, le *Mycobacterium leprae*, devenait résistant à ce médicament. Dans ces cas d'autres médicaments à base d'antibiotiques ou de sulfamides sont utilisés mais leur inocuité n'est pas satisfaisante.

Le Brevet US-A-4 112 123 concerne une composition alimentaire produisant peu de résidus et de fèces destinée à être utilisée dans le traitement de patients souffrant de troubles du catabolisme. Les différents composants de l'aliment sont choisis de manière que les besoins nutritionnels soient remplis et que la charge osmolaire soit faible. Ainsi les acides aminés sont fournis par les protéines de petit-lait non dénaturées obtenues par ultrafiltration. Il n'y a pas d'adjonction d'acides aminés libres, en particulier de tryptophane libre. La présence de composés de faible poids moléculaire n'est au contraire pas souhaitée puisque ceux-ci augmenteraient la charge osmolaire.

Des études récentes ont montré qu'un dérivé de la sérotonine, la désoxy-fructosylsérotonine, inhibait la DOPA-oxydase qui est l'enzyme spécifique du bacille de la lèpre, *Mycobacterium leprae*, et dont le rôle est d'incorporer les métabolites de la L-DOPA (L-dihydroxy-phénylalanine), facteur de sa croissance. Ce composé a un nouveau mode d'action contre le bacille et a l'avantage d'être un métabolite naturellement présent dans le corps humain. Le but de l'invention est de mettre à disposition un aliment équilibré qui puisse à la fois réduire les carences alimentaires des lépreux et réaliser les conditions permettant la synthèse de la désoxy-fructosylsérotonine *in vivo*.

Le produit alimentaire selon l'invention est caractérisé par le fait qu'il contient en poids de matière sèche:

a) 20 à 45% de protéines,

b) 4 à 10%, par rapport au poids des protéines, de L-tryptophane, dont 1 à 3% de L-tryptophane libre ajouté, par rapport au poids de matière sèche de l'aliment,

c) 5 à 35% de lipides, dont 5 à 20% contenant des acides gras susceptibles de se fixer sur l'albumine du sang et

d) 30 à 65% d'hydrates de carbone choisis parmi le glucose et les précurseurs de glucose, métabolisables en glucose dans l'organisme.

Il est particulièrement riche en protéines pour réduire la malnutrition protéique qui favorise la lèpre en diminuant l'immunité cellulaire. Ainsi, il contient de préférence 30 à 45% en poids de protéines, calculé sur matière sèche. Ces proteines sont de préférence celles qui contiennent 1 à 3% en poids de tryptophane. On peut citer les protéines d'origine lactique, de préférence provenant du lait écrémé, ou sous forme de caséinate de calcium. Ce peut être des protéines d'origine végétale, par exemple des protéines de soja. Une forme de présentation avantageuse de l'aliment est une poudre constituant une boisson par dissolution dans un milieux aqueux. On préfère donc utiliser la protéine sous forme d'isolat soluble en milieu aqueux à température ambiante par exemple sous forme de protéinate, de préférence comme protéinate de calcium. La protéine peut être rendue soluble dans les milieux aqueux de pH acide, par exemple sous forme d'isolat de protéines de petit-lait ou de soja et servir à la confection de boissons acidifiées.

L'aliment est supplémenté en L-tryptophane. Le taux de tryptophane libre ajouté correspond à une valeur totale (tryptophane lié à la protéine et tryptophane libre) de 4 à 10% et de préférence 6 à 8% en poids par rapport à la protéine. Un taux supérieur à 10% antagoniserait la résorption intestinale des autres acides aminés neutres (tyrosine, phénylalanine, leucine, isoleucine et valine) qui sont en compétition avec lui. Une partie du tryptophane résorbé au niveau de l'intestin (environ 80%) se fixe sur l'albumine du sang et le reste circule librement dans le sérum sanguin. La sérotonine du plasma est formée à partir du tryptophane circulant par l'action d'une hydroxylase conduisant au 5-hydroxytryptophane qui est lui-même transformé en sérotonine par l'action d'une décarboxylase. Cette transformation est quantitative dans la mesure où les enzymes sont présents. Celle-ci réagit à son tour avec le glucose du plasma pour former *in situ* la désoxy-fructosylsérotonine avec un rendement d'environ 30% observé dans des conditions expérimentales simulant la composition, la température et le pH qui y règnent. Le rendement global est d'environ 1% de la totalité du tryptophane ingéré. Ainsi la biodisponibilité de la désoxy-fructosylsérotonine nécessite une bonne résorption intestinale du tryptophane et sa libération de l'albumine sanguine à un niveau suffisant. Le L-tryptophane peut être ajouté sous forme d'aminoacide, d'ester, de sel ou encore de polymère. On préfère utiliser le L-tryptophane d'origine microbiologique.

L'expression «lipides contenant des acides gras susceptibles de se fixer sur l'albumine du sang» signifie qu'au moins une partie des lipides est choisie de manière à fournir dans le sang les acides gras ayant une affinité pour l'albumine sanguine. En effet les acides gras polyinsaturés et/ou ceux en $C_8$ à $C_{10}$ se fixent sur l'albumine sanguine préférentiellement au tryptophane. Ils ont la propriété de déplacer le tryptophane lié pour le libérer dans le sérum. L'aliment peut contenir les huiles végétales riches en acides gras polyinsaturés utilisées en nutrition, par exemple l'huile de carthame, de germe de blé, de tournesol, de coton et plus particulièrement l'huile de maïs, de soja (dépourvu de toute activité lipoxydase) et l'huile de pépins de raisin.

En variante, l'aliment peut contenir des triglycérides d'acides gras en $C_8$-$C_{10}$ appelés «triglycérides à chaîne moyenne». Les triglycérides à chaîne moyenne sont obtenus par extraction de produits naturels (Cuphea) ou à partir de graisses de coco et de palmiste par hydrolyse, fractionnement des acides gras, distillation et réestérification de ceux-ci avec le glycérol. Un mélange particulièrement approprié et facilement assimilable comprend 0 à 60% en poids d'une huile végétale mentionnée ci-dessus et 40% à 100% en poids de triglycérides à chaîne moyenne. Le mélange lipidique préféré mentionné ci-dessus constitue avantageusement 14 à 20% de l'aliment en poids de matière sèche.

Les acides gras polyinsaturés des lipides doivent être protégés de l'oxydation. A cet effet, la fraction lipidique, lorsqu'elle comporte des huiles riches en acides gras polyinsaturés, contient 0,02 à 1% en poids d'un antioxydant liposoluble autorisé en alimentation, par exemple le BHA (butylhydroxyanisole), le BHT (butylhydroxytoluène), les extraits antioxydants des végétaux ou la vitamine E (mélange de tocophérols naturels) individuellement ou en mélange. En effet, il semble que les acides gras oxydés ne soient plus capables de se fixer sur l'albumine au site du tryptophane et favorisant la croissance du bacille de la lèpre par leur caractère pro-oxydant (M. Bergel, Int. J. of Leprosy 26, 66, 1958).

La fraction lipidique contient de préférence environ 5% en poids de lécithine comme aide technologique (agent mouillant) pour éviter l'agglomération en paquets de la poudre lors de l'incorporation des lipides et faciliter la solubilisation dans l'eau du produit.

Dans une forme avantageuse, la fraction lipidique contient environ 50.000 U.I. (0,1% en poids) de vitamines A (acétate), indispensable à la vision et à la reconstitution des tissus épithéliaux.

L'aliment peut avantageusement se présenter sous forme de tablettes ou de barres à croquer d'utilisation commode ne nécessitant pas sa dilution dans un milieu aqueux au moment de l'emploi. Dans ce cas, la fraction lipidique peut contenir, également une graisse, par exemple le beurre de cacao ou un équivalent de celui-ci de préférence à raison de 15 à 35% en poids de l'aliment comme aide technologique pour faciliter le formage des tablettes ou des barres et pour améliorer leurs qualités organoleptiques.

Les hydrates de carbone sont choisis pour fournir dans le sang le glucose destiné à réagir avec la sérotonine circulante pour donner la forme protégée de celle-ci, la désoxy-fructosylsérotonine. Une source de glucose avantageuse est la maltodextrine (polydextrose). Selon le degré d'hydrolyse du polydextrose (nombre d'équivalents dextrose), on peut régler la cinétique d'absorption du glucose et obtenir un effet retard de manière à libérer le glucose dans le sang progressivement en rapport avec la sérotonine formée. On peut également utiliser un mélange de glucose et de maltodextrine peu hydrolysée, par exemple contenant environ 25% en poids de glucose et environ 75% en poids de maltodextrine à faible équivalent dextrose.

D'autres hydrates de carbone, précurseurs de glucose, peuvent également produire l'effet retard désiré en générant le glucose in vivo. On peut citer: le fructose, le lactose (qui ne peut naturellement être employé que dans les cas d'absence d'intolérance) et de préférence le saccharose.

Le glucose ingéré ou généré in vivo à partir de ses précurseurs libère l'insuline qui déprime le niveau plasmatique des acides aminés neutres autres que le tryptophane et augmente ainsi le niveau de ce dernier.

On peut y ajouter des édulcorants, par exemple la saccharine, le cyclamate ou l'aspartame et des agents d'aromatisation, par exemple le cacao, la vanilline, l'extrait de malt, les céréales maltées, les arômes, extraits ou pulpes des fruits par exemple de noix de coco, de banane, de fraise, etc.

Une forme préférée de l'aliment est une boisson cacaotée comprenant de la poudre de cacao soluble comme agent d'aromatisation. En plus de ses qualités aromatiques le cacao contient de la théobromine qui a une action stimulante susceptible de compenser les effets anorexique et sédatif légers du tryptophane.

Dans une variante avantageuse, l'aliment contient 0,25 à 0,75% en poids par rapport à la matière sèche de nicotinamide (niacine ou vitamine pp) dont le rôle est de diminuer la dégradation du tryptophane dans le foie et donc d'augmenter la concentration de tryptophane libre dans le sang.

Pour fabriquer l'aliment, on mélange intimement à sec les protéines et les hydrates de carbone, on y ajoute le L-tryptophane et éventuellement les agents aromatisants et édulcorants, on pulvérise le mélange lipidique à l'état liquide sur le mélange sec précédent sous agitation et on emballe la poudre obtenue à l'abri de l'oxygène.

On peut également mettre la poudre sous forme de tablettes ou de barres à croquer.

Pour ce faire, on peut par exemple mélanger les constituants solides et la fraction lipidique contenant une graisse préalablement fondue, par exemple, le beurre de cacao ou ses équivalents et substituts, mouler ou extruder la pâte obtenue, puis refroidir, démouler les portions ou découper le boudin. En variante, on peut compacter légèrement la poudre dans des moules et la chauffer de manière à la fritter, refroidir et démouler les portions.

Selon une autre présentation, on peut mettre la poudre sous forme de granulés par agglomération, par exemple à l'aide d'un sirop aqueux de maltodextrine ou de glucose suivie d'une élimination de l'eau ou encore par extrusion dans une boudineuse et découpage du boudin.

Sous forme de poudre ou de granulés, l'aliment peut être dissous dans un milieu aqueux, par exemple dans de l'eau ou dans du lait et être consommé comme boisson ou être mélangé tel que à la diète habituelle.

Ne contenant pratiquement pas d'humidité résiduelle, l'aliment est stable pendant au moins une année à l'entre-posage quelle que soit sa forme.

Il peut être administré à raison de 30 à 100 g/jour selon la constitution et l'état du patient et la nature du traitement (préventif ou curatif) en plusieurs prises, de préférence en deux prises. Il peut être appliqué à titre de complément alimentaire pour eméliorer l'efficacité d'un médicament anti-lèpre, par exemple en même temps que la désoxy-fructosylsérotonine.

Les exemples suivants illustrent l'invention. Les pourcentages et parties sont exprimés en valeur pondérale.

*Exemple 1*

On mélange 4400 g d'un concentrat de protéines de lactoserum obtenu par ultrafiltration de petit-lait doux, concentration et séchage du rétentat contenant 70% de protéines (le reste étant essentiellement constitué de lactose et contenant des sels minéraux et de la graisse lactique), avec 4000 g de maltodextrine à 40 équivalents dextrose. On ajoute 500 g de poudre de cacao soluble, 123 g de L-tryptophane d'origine microbienne, 5 g de vanilline et 10 g de saccharinate de sodium en mélangeant. On pulvérise un mélange lipidique liquide contenant 950 g d'huile de pépins de raisin, 50 g de lécithine de soja et 300 mg de butylhydroxyanisole (BHA) tout en agitant à la température ambiante. Quand le mélange (10 kg) est bien homogène, on remplit des sachets de 500 g étanches à l'air avec la poudre obtenue, on met les sachets sous azote et on les ferme hermétiquement. La poudre a la composition suivante:

| | % en poids |
|---|---|
| Protéines: protéines de lactosérum | 31 |
| dont L-tryptophane lié | (0,77) |
| L-tryptophane libre (d'origine microbienne) | 1,23 |
| Hydrates de carbone: Maltodextrine (à 40 équivalents dextrose) | 40 |
| Lipides: huile de pépins de raisin | 9,5 |
| dont acide linoléique | (7,05) |
| Divers: autres matières grasses (graisse lactique, lécithine) et autres hydrates de carbone (lactose), agents édulcorants et aromatisants, sels minéraux, antioxydant | 18,27 |

On obtient une boisson cacaotée par dissolution de 15 g de poudre dans 85 g d'eau ou de lait.

*Exemple 2*

On procède comme à l'exemple 1 à la fabrication d'une poudre cacaotée soluble dans les milieux aqueux dont les protéines sont apportées sous forme de caséinate de calcium. Celle-ci a la composition suivante:

| | % en poids |
|---|---|
| Protéines: caseine | 41,8 |
| dont L-tryptophane lié | (0,6) |
| L-tryptophane libre (d'origine microbienne) | 1,4 |

| | % en poids |
|---|---|
| Hydrates de carbone: maltodextrine (à 40 équivalents dextrose) | 40 |
| Lipides: huile de pépins de raisin | 9,5 |
| dont acide linoléique | (7,05) |
| Lécithine de soja | 0,5 |
| Vitamine E (sous forme d'acétate) | 0,1 |
| BHA (30 parties par million) | — |
| Divers: | |
| Poudre de cacao | 5 |
| Edulcorant | 0,1 |
| Aromatisant (500 parties par million) | — |
| Calcium (sous forme de caséinate) | 1,8 |

On obtient une boisson cacaotée par dissolution de 15 g de poudre dans 85 g d'eau ou de lait.

*Exemple 3*

On procède comme à l'exemple 2 à la préparation d'une poudre cacaotée soluble dans les milieux aqueux dont les hydrates de carbone sont constitués d'un mélange de maltodextrine et de glucose et les lipides d'un mélange d'huile de soja et de triglycérides à chaîne moyenne. Celle-ci a la composition suivante:

| | % en poids |
|---|---|
| Protéines: | |
| Caséine (sous forme de caséinate) | 41,8 |
| dont L-tryptophane lié | (0,54) |
| L-tryptophane libre (d'origine microbienne) | 1,97 |
| Hydrates de carbone: | |
| Maltodextrine (à 7 équivalents dextrose) | 23,9 |
| Glucose | 10 |
| Lipides: | |
| Huile de soja (dépourvu de toute activité lypoxydase) | 7 |
| dont acide linoléique | (3,6) |
| Triglycérides à chaîne moyenne | 7,5 |
| Lécithine | 0,5 |
| Vitamine E (acétate) | 0,1 |
| Divers: | |
| Aromatisants et édulcorants: poudre de cacao soluble, vanilline, saccharinate de sodium | 4,9 |
| Calcium (sous forme de caséinate) | 2,2 |
| Vitamine A (acétate) | 0,1 |
| Antioxydant (BHA) | 0,03 |

On obtient une boisson par dissolution de 15 g de poudre dans 85 g d'eau ou de lait.

*Exemple 4*

On prépare une poudre cacaotée soluble dans les milieux aqueux comme à l'exemple 2 dont la composition est la suivante:

|  | % en poids |
|---|---|
| Protéines: | |
| Caséine (sous forme de caséinate) | 41,8 |
| dont L-tryptophane lié | (0,54) |
| L-tryptophane libre (d'origine microbienne) | 1,97 |
| Hydrates de carbone: | |
| Maltodextrine (à 40 équivalents dextrose) | 33,3 |
| Lipides: | |
| Huile de pépins de raisin | 7 |
| dont acide linoléique | (5,2) |
| Triglycérides à chaîne moyenne | 7,5 |
| Lécithine | 0,5 |
| Vitamine E (acétate) | 0,1 |
| Divers: | |
| Aromatisants et édulcorants (dont 5% de poudre de cacao), calcium, antioxydant (BHA) | 7,23 |
| Nicotinamide (niacine ou vitamine PP) | 0,6 |

On obtient une boisson par dissolution de 15 g de poudre dans 85 g d'eau ou de lait.

*Exemples 5-6*

On prépare une poudre cacaotée comme à l'exemple 2 dont la composition est la suivante:

|  | % en poids |
|---|---|
| Protéines: | |
| Caséine (sous forme de caséinate) | 21 |
| dont L-tryptophane lié | (0,27) |
| L-tryptophane libre (d'origine microbienne) | 2,23 |
| Hydrates de carbone: | |
| Maltodextrine (à 40 équivalents dextrose) | 58,34 |
| Lipides: | |
| Huile de pépins de raisin | 5 |
| dont acide linoléique | (3,7) |
| Triglycérides à chaîne moyenne | 5 |
| Lécithine | 0,5 |
| Vitamine E (acétate) | 0,1 |
| Divers: | |
| Comme à l'exemple 4 sauf que la poudre de cacao est remplacée par la même quantité d'une poudre à base de cacao et de céréales maltées | 7,83 |

*Exemple 5*

On place la poudre dans des moules comportant des alvéoles de forme parallélépipédique, on la compacte légèrement sous une pression de 1 à 8 kg/cm$^2$ et on fait cheminer les moules pendant 4 min. dans un four-tunnel maintenu à 150°C puis on démoule les barres et on les refroidit.

*Exemple 6*

On met la poudre ci-dessus sous forme de granulés expansés par extrusion sous pression de la masse rendue thermoplastique, qui est expansée et découpée par des couteaux rotatifs à la sortie de la tête d'extrusion située dans une enceinte sous vide. Ces granulés peuvent être consommés tels quels ou dissous dans l'eau ou le lait.

*Exemples 7-8*

On prépare une poudre comme à l'exemple 2 dont une partie des protéines et des hydrates de carbone sont apportées sous forme de poudre de lait écrémé et dont la composition est la suivante:

|  | % en poids |
|---|---|
| Protéines: | |
| Fournies par la poudre de lait écrémé | 14,4 |
| dont L-tryptophane lié | (0,26) |
| Caséine (sous forme de caséinate) | 23,9 |
| dont L-tryptophane lié | (0,36) |
| L-tryptophane libre (d'origine microbienne) | 1,7 |
| Hydrates de carbone: | |
| Lactose apporté par la poudre de lait écrémé | 20,5 |
| Saccharose | 19,5 |
| Lipides: | |
| Triglycérides à chaîne moyenne | 7,5 |
| Lécithine | 0,5 |
| Divers: | |
| Autres matières grasses (graisse lactique résiduelle), agent aromatisant (5% de poudre de cacao ou 5% d'arôme de fraise sur maltodextrine), Nicotinamide (0,6%), sels minéraux. | 11,9 |

*Exemple 7*

On obtient une boisson par dissolution de 15 g la poudre ci-dessus dans 85 g d'eau ou de lait.

*Exemple 8*

On mélange 100 parties de la poudre ci-dessus et 20 parties de beurre de cacao ou d'équivalent de beurre de cacao préalablement fondu à 45°C dans un malaxeur. On remplit les alvéoles parallélépipédiques d'un moule avec la pâte coulante obtenue, on refroidit les moules à une température inférieure à 37°C, on démoule les articles et on les emballe dans du papier d'aluminium.

*Exemple 9-10*

On prépare une poudre comme à l'exemple 2 dont une partie des protéines et des hydrates de carbone sont fournies sous forme de concentrat de soja (à 70%) de protéines et dont la composition est la suivante:

|  | % en poids |
|---|---|
| Protéines: | |
| Fournies par le concentrat de soja | 38,1 |
| dont L-tryptophane lié | (0,51) |
| L-tryptophane libre (d'origine microbienne) | 1,9 |
| Hydrates de carbone: | |
| Fournis par le concentrat de soja | 10,9 |
| Saccharose | 30 |
| Lipides: | |
| Triglycérides à chaîne moyenne | 7,5 |
| Lécithine | 0,5 |
| Divers: | |
| Autres matières grasses (provenant du concentrat de soja), agent aromatisant (5% de poudre de cacao ), Nicotinamide (0,6%), sels minéraux. | 11,1 |

*Exemple 9*

On obtient une boisson cacaotée par dissolution de 15 g de la poudre ci-dessus dans 85 g d'eau ou de lait.

*Exemple 10*

En procédant comme à l'exemple 8 à partir de 100 parties de poudre ci-dessus et de 20 parties d'un substitut de beurre de cacao (stéarine de coco ou de palmiste), on obtient des barres à croquer.

*Exemples 11-12*

On prépare une poudre comme à l'exemple 2 dont les protéines proviennent d'un isolat de soja à 90% de protéines et dont la composition est la suivante:

|  | % en poids |
|---|---|
| Protéines: | |
| Isolat de soja | 38,1 |
| dont L-tryptophane lié | (0,52) |

|  | % en poids |
|---|---|
| L-tryptophane libre (d'origine microbienne) | 1,9 |
| Hydrates de carbone: | |
| Saccharose | 42,1 |
| Lipides: | |
| Triglycérides à chaîne moyenne | 7,5 |
| Lécithine | 0,5 |
| Divers: | |
| Agent aromatisant (5% de poudre de cacao), Nicotinamide (0,6%), sels minéraux. | 9,9 |

*Exemple 11*

On obtient une boisson cacaotée par dissolution de 15 g de la poudre ci-dessus dans 85 g d'eau ou de lait.

*Exemple 12*

En procédant comme à l'exemple 8 à partir de 100 parties de poudre ci-dessus et de 20 parties de beurre de cacao on obtient des barres à croquer.

*Exemple 13*

On a montré l'efficacité de l'aliment de l'exemple 2 dans le traitement de la lèpre en utilisant le test de prolifération de *M. Leprae* sur les coussinets de souris inoculés avec des bacilles d'origine humaine selon C.C. Shepard, 1960 (J. Exp. Med. 112, 445). On a administré pendant un an 0,5 g d'aliment par jour aux souris albinos, dont le coussinet plantaire a été inoculé par $10^4$ bacilli de *M. leprae*, provenant de la biopsie de 5 malades LL (infection multibacillaire, état lépromateux) avec I. M. (indice morphologique) entre 1 et 2% et I. B. (indice bactériologique) minimum 2%. L'un de ces malades s'est avéré résistant au 4,4'-diamino-diphénylsulfone (DDS). L'efficacité de l'aliment a été comparée à l'effet de 0,01% de DDS dans la nutrition des souris (soit 20 mg de DDS/kg).

Chaque groupe a été constitué par 10 souris et les valeurs présentées dans le tableau ci-après correspondent à la moyenne des 10 niveaux de *M. leprae*: pour évaluer les résultats, on a compté le nombre de bacilles viables à l'aide d'un microscope dans les champs 20-22 sur les taches des coussinets des pattes de souris.

| Cas | Traitement | 6 mois | 8 mois | 10 mois | 12 mois |
|---|---|---|---|---|---|
| No. 1 | DDs | neg.[a] | neg. | neg. | neg |
|  | Aliment |  | neg. | neg. | neg. |
| No. 2 | DDS | neg. | neg. | neg. | neg |
|  | Aliment |  | neg. | neg. | neg. |
| No. 3 | DDS | 22x[b] | 39x | 20x | 49x |
|  | Aliment |  |  | 27x | 40x |
| No. 4 | DDS | neg. | neg. | neg. | neg. |
|  | Aliment | neg. | neg. | neg. | neg. |
| No. 5 | DDS | neg. | neg. | neg. | neg. |
|  | Aliment |  | neg. | neg. | neg. |

*Legende:* a neg. signifie qu'on n'a pas pu détecter de bacille *M. Leprae* viable dans les champs 20-22 du microscope dans chacune des taches.

b ...x signifie ... fois plus de bacilles *M. leprae* comptés qu'au début de l'expérience.

Les résultats ci-dessus montrent que le présent aliment a été au moins aussi efficace que le médicament dans le cas des bacilles non-résultants au DDS. Dans le cas 3, de bacilles résistants au DDS on a observé une diminution sensible de la prolifération par comparaison avec le contrôle traité au DDs.

**Revendications**

1. Produit alimentaire efficace dans le traitement de la lèpre caractérisé par le fait qu'il comprend en poids de matière sèche:

   a) 20 à 45% de protéines,
   b) 4 à 10%, par rapport au poids des protéines, de L-tryptophane, dont 1 à 3% de L-tryptophane libre ajouté, par rapport au poids de matière sèche de l'aliment,
   c) 5 à 35% de lipides, dont 5 à 20% contenant des acides gras susceptibles de se fixer sur l'albumine du sang et
   d) 30 à 65% d'hydrates de carbone choisis parmi le glucose et les précurseurs de glucose, métabolisables en glucose dans l'organisme.

2. Produit alimentaire selon la revendication 1, caractérisé par le fait qu'il comprend, en poids de matière sèche 30 à 45% de protéines, de préférence sous forme de caséinate de calcium ou provenant du lait écrémé ou du soja.

3. Produit alimentaire selon l'une des revendications 1 et 2, caractérisé par le fait que les lipides contenant des acide gras susceptibles de se fixer sur l'albumine du sang sont constitués de 0 à 60% en poids d'une huile végétale riche en acides gras polyinsaturés et de 40 à 100% en poids de triglycérides d'acides gras en $C_8$-$C_{10}$ et qu'ils sont protégés contre l'oxydation par addition de 0,02 à 1% en poids d'un antioxydant liposoluble.

4. Produits alimentaire selon la revendication 1, caractérisé par le fait que les hydrates de carbone sont principalement le glucose et la maltodextrine.

5. Produit alimentaire selon la revendication 1, caractérisé par le fait qu'il contient 0,25 à 0,75% en poids de matière sèche de nicotinamide.

6. Produit alimentaire selon la revendication 1, caractérisé par le fait qu'il se présente sous forme de poudre ou de granulés solubles dans les milieux aqueux constituant une boisson par dissolution dans lesdits milieux aqueux, ou sous forme de tablette ou de barre à croquer.

7. Procédé de préparation d'un produit alimentaire efficace dans le traitement de la lèpre, caractérisé par le fait qu'un mélange intimement à sec des protéines et des hydrates de carbone, on y ajoute du L-tryptophane et éventuellement des agents aromatisants et édulcorants et qu'on pulvérise un mélange lipidique à l'état liquide sur le mélange sec précédent sous agitation de manière à obtenir une poudre comprenant en poids de matière sèche:

   a) 20 à 45% de protéines,
   b) 4 à 10%, par rapport au poids des protéines, de L-tryptophane, dont 1 à 3% de L-tryptophane libre ajouté, par rapport au poids de matière sèche de l'aliment,

   c) 5 à 35% de lipides, dont 5 à 20% contenant des acides gras susceptibles de se fixer sur l'albumine du sang et 30 à 65% d'hydrates de carbone choisis parmi le glucose et les précurseurs de glucose, métabolisables en glucose dans l'organisme.

8. Procédé selon la revendication 7, caractérisé par le fait qu'on compacte la poudre obtenue sous forme de tablettes ou de barres ou qu'on l'agglomère sous forme de granulés.

**Patentansprüches**

1. Zur Behandlung von Lepra wirksames Nahrungsmittel, dadurch gekennzeichnet, dass es im Gewicht der Trockenmasse enthält:

   a) 20 bis 45% Proteine,
   b) 4 bis 10%, bezogen auf das Gewicht der Proteine, L-Tryptophan, davon 1 bis 3%, bezogen auf das Gewicht der Trockenmasse des Nahrungsmittels, freies zugesetztes L-Tryptophan,
   c) 5 bis 35% Lipide, davon ein Gehalt von 5 bis 20% Fettsäuren, die vom Albumin des Blutes fixierbar sind, und
   d) 30 bis 65% Kohlenhydrate, die ausgewählt sind aus Glucose und Glucosevorläufern, die im Organismus zu Glucose metabolisiert werden.

2. Nahrungsmittelprodukt nach Anspruch 1, dadurch gekennzeichnet, dass es, bezogen auf das Gewicht der Trockenmasse, 30 bis 45% Proteine enthält, vorzugsweise in der Form von Calciumcaseinat, aus entrahmter Milch oder aus Soja.

3. Nahrungsmittelprodukt nach einem der Ansprüche 1 und 2, dadurch gekennzeichnet, dass die Lipide, die vom Albumin des Blutes fixierbare Fettsäuren enthalten, gebildet werden von 0 bis 60 Gew.-% eines Pflanzenöls, das reich an mehrfach ungesättigten Fettsäuren ist, und von 40 bis 100 Gew.-% von Triglyceriden von $C_8$-$C_{10}$ Fettsäuren, und gegen eine Oxidation durch Zugabe von 0,02 bis 1 Gew.-% eines lipidlöslichen Antioxidans geschützt sind.

4. Nahrungsmittelprodukt nach Anspruch 1, dadurch gekennzeichnet, dass die Kohlenhydrate hauptsächlich Glucose und Maltodextrin sind.

5. Nahrungsmittelprodukt nach Anspruch 1, dadurch gekennzeichnet, dass es 0,25 bis 0,75 Gew.-% der Trockenmasse an Nicotinamid enthält.

6. Nahrungsmittelprodukt nach Anspruch 1, dadurch gekennzeichnet, dass es in Form von in einem wässrigen Milieu löslichem Pulver oder Granulat vorliegt, die bei der Auflösung in diesem wässrigen Milieu ein Getränk ergeben, oder in Form einer Tablette oder einer Knabberstange.

7. Verfahren zur Herstellung eines zur Behandlung von Lepra wirksamen Nahrungsmittelprodukts, dadurch gekennzeichnet, dass man eine innige trockene Mischung der Proteine und der Kohlehydrate herstellt, L-Tryptophan und gegebenenfalls Aromatisierungsmittel und Süssstoffe zusetzt und dass man eine Lipidmischung im flüssigen Zustand auf die genannte trockene Mischung aufsprüht und diese dabei bewegt, so dass man ein Pulver erhält, das in Gewicht der Trockenmasse enthält:

a) 20 bis 45% Proteine,

b) 4 bis 10%, bezogen auf das Gewicht der Proteine, L-Tryptophan, davon 1 bis 3%, bezogen auf das Gewicht der Trockenmasse des Nahrungsmittels, freies zugesetztes L-Tryptophan,

c) 5 bis 35% Lipide, davon ein Gehalt von 5 bis 20% Fettsäuren , die vom Albumin des Blutes fixierbar sind, und

d) 30 bis 65% Kohlenhydrate, die ausgewählt sind aus Glucose und Glucosevorläufern, die im Organismus zu Glucose metabolisiert werden.

8. Verfahren nach Anspruch 7, dadurch gekennzeichnet, dass man das erhaltene Pulver in Tabletten- oder Stangenform verpresst oder dass man es zu einem Granulat agglomeriert.

**Claims**

1. A food product effective in the treatment of leprosy, characterized in that it comprises by weight of dry matter:

a) from 20 to 45% of proteins,

b) from 4 to 10%, by weight of the proteins, of L-tryptophan, of which from 1 to 3% is added free L-tryptophan, by weight of dry matter of the food product,

c) from 5 to 35% of lipids and from 5 to 20% of lipids containing fatty acids capable of fixing themselves to the albumin in the blood and

d) from 30 to 65% of carbohydrates selected from glucose and glucose precursors which are metabolizable in glucose in the organism.

2. A food product as claimed in claim 1, characterized in that it comprises from 30 to 45% by weight, based on dry matter, of proteins, preferably in the form of calcium caseinate, or from skimmed milk or soybean.

3. A food product as claimed in any preceding claims, characterized in that the lipids containing fatty acids capable of fixing themselves to the albumin in the blood consists of from 0 to 60% by weight of a vegetable oil rich in polyunsaturated fatty acids and from 40 to 100% by weight of $C_8$-$C_{10}$ fatty acid and triglycerides, and that the lipids are protected against oxidation by the addition of from 0.02 to 1% by weight of a liposoluble antioxydant.

4. A food product as claimed in claim 1, characterized in that the carbohydrates are essentially glucose and maltodextrin.

5. A food product as claimed in claim 1, characterized in that it contains from 0.25 to 0.75% by weight of nicotinamide, based on dry matter.

6. A food product as claimed in claim 1, characterized in that it is in the form of a powder or granulates soluble in aqueous media constituting a beverage by dissolution in those aqueous media, or in the form of a tablet or bar to be crunched.

7. A process for producing a food product effective in the tretment of leprosy, characterized in that proteins and carbohydrates are thoroughly dry-mixed, L-tryptophan and, optionally, flavouring and sweetening agents are added and a lipidic mixture is sprayed in the liquid state onto the resulting mixture so as to obtain a product containing by weight of dry matter:

a) from 20 to 45% of proteins,

b) from 4 to 10%, by weight of the proteins, of L-tryptophan, of which from 1 to 3% is added free L-tryptophan, by weight of dry matter of the food product,

c) from 5 to 35% of lipids, and from 5 to 20% of lipids containing fatty acids capable of fixing themselves to the albumin in the blood and

d) from 30 to 65% of carbohydrates selected from glucose and glucose precursors which are metabolizable in glucose in the organism.

8. A process as claimed in claim 7, characterised in that the powder obtained is compacted in the form of tablets or bars or is agglomerated in the form of granulates.